(19) Europäisches Patentamt
European Patent Office
Office européen des brevets

(11) EP 4 657 443 A1

(12) EUROPEAN PATENT APPLICATION
published in accordance with Art. 153(4) EPC

(43) Date of publication:
03.12.2025 Bulletin 2025/49

(21) Application number: 23918753.7

(22) Date of filing: 20.11.2023

(51) International Patent Classification (IPC):
G16B 20/20 (2019.01)     G16B 30/10 (2019.01)
G16B 40/20 (2019.01)     G16B 20/30 (2019.01)
G16B 10/00 (2019.01)     G16B 45/00 (2019.01)
G06N 3/02 (2006.01)      G06N 20/00 (2019.01)

(52) Cooperative Patent Classification (CPC):
G06N 3/02; G06N 20/00; G16B 10/00; G16B 20/20;
G16B 20/30; G16B 30/10; G16B 40/20;
G16B 45/00

(86) International application number:
PCT/KR2023/018626

(87) International publication number:
WO 2024/158118 (02.08.2024 Gazette 2024/31)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 26.01.2023 KR 20230009883

(71) Applicant: Genomecare Co., Ltd
Suwon-si, Gyeonggi-do 16229 (KR)

(72) Inventors:
• KIM, Sunshin
Yongin-si Gyeonggi-do 17003 (KR)
• ADHIKARI, Krishna Prasad
Hwaseong-si Gyeonggi-do 18261 (KR)
• JEONG, Jae Hwan
Suwon-si Gyeonggi-do 16213 (KR)
• OH, Gyeong In
Seoul 07051 (KR)

(74) Representative: ABG Intellectual Property Law,
S.L.
Avenida de Burgos, 16D
Edificio Euromor
28036 Madrid (ES)

(54) **METHOD FOR DETECTING FETAL COPY NUMBER VARIATIONS BASED ON VIRTUAL POSITIVE AND NEGATIVE DATA**

(57)    The present invention provides a method for detecting fetal copy number variations (CNVs) based on synthetic positive and negative data, as well as a computer-readable medium recording a program applied to perform this method. Accordingly, the present invention allows for non-invasive prenatal diagnosis of fetal CNVs with high sensitivity and specificity.

EP 4 657 443 A1

Figure 1

Fetal DNA fraction ratio (FF) :10%

Size of 1p36 overall region
(0 Mbp ~ 28 Mbp)
320,000 reads

0Mbp 1Mbp

8Mbp

28Mbp

Random removal of 4,000 reads
corresponding to 5% fetal DNA
fraction (FF) from 80,000 reads
of any target site (1 Mbp ~ 8 Mbp)

## Description

## Technical Field

The present invention provides a method for detecting fetal copy number variations based on synthetic positive and negative data, i.e., synthetic copy number variation (CNV) data and synthetic normal data.

## Background Art

Prenatal diagnosis refers to diagnosing the presence or absence of a disease in the fetus before the fetus is born. Prenatal diagnosis is largely divided into invasive and non-invasive diagnostic methods. Invasive diagnostic methods include, for example, chorionic villus sampling, amniocentesis, and umbilical cord blood sampling. Invasive diagnostic methods have the potential to cause miscarriage, disease, or deformity by causing shock to the fetus during the test process, and thus non-invasive diagnostic methods that are performed prior to the invasive methods are being developed.

Recently, it has been demonstrated that non-invasive diagnosis of fetal copy number variations is feasible by massive parallel sequencing of DNA molecules in the plasma of pregnant women. Fetal DNA can be detected in maternal plasma and serum from the 7th week of pregnancy, and the amount of fetal DNA in maternal blood increases with the period of pregnancy. However, since the prevalence of fetuses with copy number variations is very low, it is very difficult to obtain positive data, making it difficult to develop detection technology for test samples. In addition, even if positive data are obtained, there is a problem that the sensitivity and specificity of copy number variation detection are low because the threshold for distinguishing a normal fetus from a fetus with copy number variations is unclear when performing massive parallel sequencing of fetal DNA with low sequencing coverage or when the fetal DNA fraction is low.

Therefore, there is a need to develop a method that can clearly distinguish a normal fetus from a fetus with copy number variations and increase the sensitivity and positive predictive value of copy number variation detection by overcoming these limitations.

## Prior Art Document

## Patent Document

(Patent Document 1) KR 2031841 B

## Detailed Description of Invention

## Technical Problem

The present invention provides a method for detecting fetal copy number variations.

The present invention provides a computer-readable medium recording a program applied to perform the above method.

## Solution to Problem

In one aspect, there is provided a method for detecting fetal copy number variations, comprising:

obtaining sequence information (read) of a plurality of nucleic acid fragments obtained from a biological sample of a pregnant woman with a normal fetus;
mapping the obtained sequence information to a human reference genome and aligning the sequence information of the nucleic acid fragments to a chromosome;
calculating the read count (RC), fetal DNA fraction (fetal fraction: FF), and GC content of the sequence information of the nucleic acid fragments in the target chromosome in a normal fetus, based on the sequence information of the nucleic acid fragments aligned to the chromosome;
producing synthetic normal data from normal data based on the read count and fetal DNA fraction in the target chromosome in a normal fetus, wherein the normal data is the read count in the target chromosome site in a normal fetus;
producing synthetic positive data from the synthetic normal data, wherein the synthetic positive data is the read count in the target chromosome site in a fetus with copy number variations;
establishing a copy number variation discrimination model from the produced synthetic normal data and synthetic positive data; and
detecting copy number variations of a test sample using the copy number variation discrimination model.

The term "copy number variation (CNV)" refers to a genetic change corresponding to a structural variation in individual variation of a genome. The copy number variation may be a condition in which the number of chromosome parts per cell in a cell, individual, or lineage is not an integer multiple of the basic number, and is one to several more or less than the integer multiple, i.e., a condition involving a genome with an incomplete structure. The copy number variation may be a case in which two or only one of a pair of homologous chromosomes is deleted in the case of a diploid, or a case in which another extra chromosome part is present and is duplicated in addition to a pair of homologous chromosomes.

The method comprises obtaining sequence information (read) of a plurality of nucleic acid fragments obtained from a biological sample of a pregnant woman with a normal fetus.

The pregnant woman may be a pregnant woman with a single fetus or multiple fetuses (e.g., twin fetuses).

The biological sample may be blood, plasma, serum, urine, saliva, mucosal secretions, sputum, feces, tears, or a combination thereof. The biological sample is, for example, peripheral blood plasma. The biological sample may include a nucleic acid of a fetus. The nucleic acid of a fetus may be cell-free DNA (cfDNA). The nucleic acid of a fetus may be isolated DNA.

The obtaining sequence information of a plurality of nucleic acid fragments obtained from a biological sample of a pregnant woman may comprise isolating nucleic acids from a biological sample.

The obtaining sequence information may include isolating cell-free DNA (cfDNA) from the biological sample. The method of isolating nucleic acids from the biological sample may be performed by methods known to those of ordinary skill in the art. A length of the isolated nucleic acid fragment may be about 10 bp (base pair) to about 2000 bp, about 15 bp to about 1500 bp, about 20 bp to about 1000 bp, about 20 bp to about 500 bp, about 20 bp to about 200 bp, or about 20 bp to about 100 bp.

The obtaining sequence information of a plurality of nucleic acid fragments from a biological sample obtained from a pregnant woman may include performing massive parallel sequencing on the isolated nucleic acids.

The term "massive parallel sequencing" may be used interchangeably with nextgeneration sequencing (NGS) or second-generation sequencing. Massive parallel sequencing refers to a technique for simultaneously sequencing millions of fragments of nucleic acids. Massive parallel sequencing may be performed in parallel, for example, by 454 Platform (Roche), GS FLX Titanium, Illumina MiSeq, Illumina HiSeq, Illumina Genome Analyzer, Solexa platform, SOLiD System (Applied Biosystems), Ion Proton (Life Technologies), Complete Genomics, Helicos Biosciences Heliscope, single molecule real-time (SMRT™) technology from Pacific Biosciences, or a combination thereof.

The method may further comprise preparing a nucleic acid library in order to perform massive parallel sequencing.

The nucleic acid library may be prepared according to the method of massive parallel sequencing. The nucleic acid library may be constructed according to the manufacturer's instructions that provide massive parallel sequencing.

The obtained sequence information of the nucleic acid fragments may also be referred to as a read. The obtained sequence information of the nucleic acid fragments may have a certain range of sequencing coverage. The sequencing coverage may also be referred to as sequencing depth. The sequencing coverage may be the average number of times of sequence information that is sequenced in a sequence reconstructed through massive parallel sequencing. The sequencing coverage may be calculated by the mathematical equation of (length of reads x number of reads) / haploid genome length. The above sequencing coverage may be from about 0.00001 to about 1, from about 0.0001 to about 0.5, from about 0.001 to about 0.1, from about 0.001 to about 0.05, from about 0.003 to about 0.05, from about 0.005 to about 0.05, from about 0.007 to about 0.04, from about 0.01 to about 0.03, from about 0.015 to about 0.03, from about 0.02 to about 0.03, from about 0.025 to about 0.03, from about 0.01 to about 0.025, from about 0.01 to about 0.02, or from about 0.01 to about 0.015.

The method comprises mapping the obtained sequence information to a human reference genome and aligning the sequence information of the nucleic acid fragments to a chromosome.

The human reference genome may be hg18 or hg19. The sequence information that is mapped to only one genomic location in a human reference genome may be aligned as unique sequence information. Based on the aligned unique sequence number, the sequence information of the nucleic acid fragments may be aligned to the location of the chromosome. The location of the chromosome may be a continuous range on a chromosome having a length of about 5 kb or more, about 10 kb or more, about 20 kb or more, about 50 kb or more, about 100 kb or more, about 1000 kb or more, or 2000 kb or more. The location of the chromosome may be a single chromosome.

After aligning the sequence information of the nucleic acid fragments to a chromosome, the method may further comprise checking the depth distribution of the sequence information of the nucleic acid fragments aligned to the chromosome for each section and excluding sections with low reliability of the sequence information from the analysis target. The section may be a section set in a unit of about 5 kb to about 50 kb. For example, the section may be a section set to about 10 kb to about 40 kb, about 15 kb to about 30 kb, or about 20 kb to about 25 kb. By setting the above section, filtering may be done using the GC content of the nucleotide sequence. In addition, by setting the above section, a group of the depth and GC content of the sequence information of the nucleic acid fragments aligned to the chromosome may be form, and statistical analysis may be performed.

The excluding sections with low reliability of the sequence information from the analysis target may include removing mismatch portions, removing the sequence information aligned to multiple sites, removing duplicated sequence information, or a combination thereof. In order to exclude sections with low reliability of the sequence information from the analysis target, quality filtering, trimming, perfect match, removal of sequences aligned to multiple locations, removal of PCR duplicated sequence information (PCR duplicated reads), or a combination thereof may be performed. The quality filtering is a process of extracting sequence information with high quality for the quality of each nucleotide sequence obtained during the sequencing process. The trimming is a process of removing parts of poor quality because the quality of the latter part of the nucleotide sequence is low due to the nature of the sequencing device. For example, nucleic acid fragments may be trimmed to a size of at least about 50 bp, greater than about 50 bp, or greater than about 100 bp. For example, the quality value of the nucleic acid fragment may be 20 or higher, 30 or higher, 40 or higher, or 50 or higher. The perfect match selects only nucleotide sequences that perfectly match when mapping to a human reference genome. Since sequences aligned to multiple locations are likely to be repetitive sequence regions, sequences aligned to multiple locations may be removed from the obtained sequence information. Removing the PCR duplicated sequence information means removing parts that have been amplified more due to errors during the sequencing process. In addition, for statistical analysis, a group with a certain degree of even deviation must be selected to obtain meaningful results. The part without depth is usually the N-region of the chromosome and may be excluded from the analysis target.

The method comprises calculating the read count (RC), fetal DNA fraction (fetal fraction: FF), and GC content of the sequence information of the nucleic acid fragments for a specific portion of the target chromosome in a normal fetus, based on the sequence information of the nucleic acid fragments aligned to the chromosome.

The target chromosome refers to a chromosome that is targeted or to be detected. The chromosome refers to a thick thread-like or rod-shaped structure that appears in the nucleus during cell division, and refers to a structure in which the DNA and histone proteins of a living organism are condensed. The chromosome refers to both a loose structure and a condensed structure. In the case of humans, they are diploid, and have 22 autosomes, 2 of which exist as homologous chromosomes, and have sex chromosomes, either two chromosome X (female) or one chromosome X and one chromosome Y (male).

The specific portion of the target chromosome may be selected from the group consisting of chromosome 1 to chromosome 22, chromosome X, and chromosome Y.

The term "read count (RC)" or "read number" is a value that counts the number of reads aligned to the location of one gene.

The term "fetal DNA fraction (fetal fraction: FF)" or "fraction of fetal nucleic acids" refers to the amount or ratio (%) of fetal nucleic acids among nucleic acids isolated from a biological sample of a pregnant woman. The fetal fraction may be a concentration, relative ratio, or absolute amount of fetal nucleic acids. The fetal nucleic acid may be a nucleic acid derived from fetal placenta trophoblasts.

The term "GC content" refers to the ratio (%) of guanine (G) and cytosine (C) among the bases that make up DNA. The GC content may be calculated by the equation of GC content = (G+C) / (A+T+G+C).

The method comprises producing synthetic normal data from normal data based on the read count and fetal DNA fraction in the target chromosome in a normal fetus.

The normal data may be the read count in the target chromosome site in a normal fetus. The normal data may also be referred to as negative data.

The target chromosome site refers to a site or region of a chromosome that is targeted or to be detected. The target chromosome site may be a site of one selected from the group consisting of chromosome 1 to chromosome 22, chromosome X, and chromosome Y. The target chromosome site may be a site of one selected from the group consisting of chromosome 1, chromosome 5, chromosome 8, chromosome 9, and chromosome 17. The target chromosome site may be selected from the group consisting of chromosome 1 short arm 36 site (1p36), chromosome 5 short arm site (5p), chromosome 8 short arm 23.1 site (8p23.1), chromosome 9 short arm site (9p), and chromosome 17 long arm 21.31 site (17q21.31).

The synthetic normal data may be a read count of a target chromosome obtained by randomly combining and dividing normal data.

The method comprises producing synthetic positive data from the synthetic normal data.

The synthetic positive data may be the read count in the target chromosome site in a fetus with copy number variations. The synthetic positive data may be the synthetic read count obtained from the synthetic normal data.

The copy number variation may be any one variation selected from the group consisting of insertion, deletion, duplication, inversion, and translocation. The insertion refers to a chromosomal abnormality in which a part of a chromosome is added. The deletion refers to a chromosomal abnormality in which a part of a chromosome is deleted. The duplication refers to a chromosomal abnormality in which a part of a chromosome is added and a part of a chromosome is doubled. The inversion refers to a chromosomal abnormality in which a part of a chromosome is broken and then arranged inverted. The translocation refers to a chromosomal abnormality in which a part of a chromosome is broken and attached to a chromosome other than a homologous chromosome. The chromosomal abnormality may be a chromosomal mutation in which an abnormality occurs in the number or structure of chromosomes.

The copy number variation may be selected from the group consisting of 1p36 deletion, 5p deletion, 8p23.1 duplication, 9p deletion, and 17q21.31 duplication.

The copy number variation may be recorded in the Orphanet (www.orpha.net/consor/cgi-bin/index.php) rare disease database, which contains information on chromosomal deletions and duplications related to more than 350 congenital genetic diseases. For example, 1p36 deletion syndrome is caused by partial deletion of a specific region (p36) of the short arm (p-arm) of chromosome 1, and has a prevalence of about 1/5,000. In addition, 5p syndrome (Cri-du-chat syndrome) is caused by partial deletion of the short arm (p-arm) of chromosome 5, and has a prevalence of about 1/15,000.

The copy number variation may cause one of the disorders selected from 1p36 deletion syndrome, 5p deletion syndrome (Cri-du-chat syndrome), 8p23.1 duplication syndrome, 9p deletion syndrome, 11q deletion syndrome (Jacobsen syndrome), 17q21.31 duplication syndrome, autism spectrum disorder, epilepsy, schizophrenia, TAR (thrombocytopenia-absent radius) syndrome, HNPP syndrome, 3q29 microdeletion syndrome, 8p23.1 deletion syndrome, Sotos syndrome, Langer-Giedion syndrome, WAGR syndrome (Wilms tumour-aniridia syndrome, aniridia-Wilms tumour syndrome), Koolen-de Vries syndrome, Beckwith-Wiedemann syndrome, DiGeorge syndrome, Charcot-Marie-Tooth disease, Miller-Dieker Lissencephaly syndrome, Angelman syndrome, Williams syndrome, Smith-Magenis syndrome, Prader-Willi syndrome, De Grouchy syndrome, Xp11.2 duplication syndrome, and Wolf-Hirschhorn syndrome, Mowat-Wilson syndrome, Mesomelia-Synostoses syndrome, Kleefstra syndrome, Kagami-Ogata syndrome, Alagille syndrome, Okihiro syndrome, Aldred syndrome, Syndactyl-Nystagmus syndrome, Silver-Russell syndrome, and Yuan-Harel-Lupski syndrome.

The producing the synthetic positive data may use the read count and FF corresponding to any specific part of the chromosome in order to detect the copy number variation. For example, in the case of 1p36, the read count and FF of the 36th region of the short arm (p-arm) of chromosome 1 are used.

The producing synthetic positive data from the synthetic normal data may be determined from the equation of DRCPC = RCPC - RCPC × FF/2 when the copy number variation of the target chromosome site is a deletion. In the above equation, DRCPC is the read count in the target chromosome site in a copy number variation fetus, RCPC is the read count in the target chromosome site in a normal fetus, and FF is the fetal DNA fraction.

The producing synthetic positive data from the synthetic normal data may be determined from the equation of DRCPC = RCPC + RCPC × FF/2 when the copy number variation of the target chromosome site is a duplication. In the above equation, DRCPC is the read count in the target chromosome site in a copy number variation fetus, RCPC is the read count in the target chromosome site in a normal fetus, and FF is the fetal DNA fraction.

In the producing synthetic positive data from the synthetic normal data, if the target chromosome is a 1p36 deletion copy number variation, it shows the p36 site of chromosome 1 and the target size corresponds to 28 Mbp (mega base pairs). In this case, the deletion size of the copy number variation randomly produces a site with a target size of 28 Mbp or less. The p36 site of chromosome 1 is divided into certain sections (e.g., 300 Kb) and the read count is calculated for each section and used to detect positive data.

The method comprises establishing a copy number variation discrimination model from the produced synthetic normal data and synthetic positive data.

The establishing a copy number variation discrimination model from the produced synthetic normal data and synthetic positive data is performed based on RCPC (read count of a part of chromosome), RCPCi (i = 1 to i = k, i; sections of chromosome site, k; the total number of sections of chromosome site), FF, and GC content, or a combination thereof, when the copy number variation of the target chromosome site is a deletion or duplication, wherein RCPC may be normal and positive data.

The copy number variation discrimination model may be trained by applying a machine learning algorithm. The machine learning algorithm may be selected from the group consisting of LGBM (Light Gradient Boosting Machine), AdaBoost, Voting Classifier, Random Forest, logistic regression analysis (logistic algorithm), artificial neural network, and QDA (Quadratic Discriminant Analysis). The LGBM may be described in Guolin Ke et al., "LightGBM: A Highly Efficient Gradient Boosting Decision Tree". Proceedings of the 31st International Conference on Neural Information Processing Systems (NIPS 2017), pp. 3149-3157.

The method comprises detecting copy number variations of a test sample using the copy number variation discrimination model.

The test sample refers to a biological sample for testing a copy number variation of a fetus. The above biological sample is the same as described above.

When the target chromosome site is 1p36 deletion, the method may be performed based on the total read count in the target chromosome site, RCPC (read count of a part of chromosome), the read count of each section of the corresponding site, RCPCi (i = 1 to k, k is the total number of sections), FF, and GC content as input values according to the corresponding site.

If a positive result is obtained by inputting the input value of the test sample into the copy number variation discrimination model, the copy number variations of the test sample may be detected.

The method may further comprise performing any one invasive method selected from the group consisting of chorionic villus sampling, amniocentesis, and umbilical cord blood sampling based on the detection results of the copy number variations of the test sample.

In another aspect, there is provided a computer-readable medium recording a program applied to perform the method for detecting fetal copy number variations according to one aspect.

The copy number variation detection method may be implemented as a program (or application) including an executable algorithm that may be executed on a computer. The program may be provided by being stored on a transitory or non-transitory computer readable medium.

The non-transitory computer readable medium refers to a medium that stores data semi-permanently and may be read by a device, rather than a medium that stores data for a short period of time, such as a register, cache, or memory. For example, the application or program may be provided by being stored in a non-transitory computer readable medium, such as a CD, DVD, hard disk, Blu-ray disk, USB, memory card, ROM (read-only memory), PROM (programmable read only memory), EPROM (erasable PROM: EPROM), EEPROM (electrically EPROM), or flash memory.

The transitory computer readable medium refers to various RAMs such as Static RAM (SRAM), Dynamic RAM (DRAM), Synchronous DRAM (SDRAM), Double Data Rate SDRAM (DDR SDRAM), Enhanced SDRAM (ESDRAM), Synclink DRAM (SLDRAM), and Direct Rambus RAM (DRRAM).

**Effects of Invention**

According to a method for detecting fetal copy number variations based on synthetic data according to one embodiment, and a computer-readable medium recording a program applied to perform this method, the present invention allows for non-invasive prenatal diagnosis of fetal CNVs with high sensitivity and specificity.

**Brief Description of Drawings**

Figure 1 shows the size of the overall region of 1p36 deletion syndrome and an example of a method for generating a copy number variation, which is positive data, from normal data for a target site of chromosome 1. The size of the overall region of 1p36 is about 28 Mbp. Here, the start point (about 1 Mbp) and the end point (about 8 Mbp) of the target site are arbitrarily determined in this overall region, and the number of reads (about 4,000, which is 5%) corresponding to half of the fetal fraction (about 10%) is removed from the total number of about 80,000 reads of the target site.

Figure 2 shows the sensitivity and positive predictive value for the learning results of 1p36 deletion syndrome, and the results verified using the model obtained from this learning are shown as test data.

Figure 3 shows the sensitivity and positive predictive value for the learning results of 5p deletion syndrome, and the results verified using the model obtained from this learning are shown as test data.

Figure 4 shows the sensitivity and positive predictive value for the learning results of 9p deletion syndrome, and the results verified using the model obtained from this learning are shown as test data.

Figure 5 shows the sensitivity and positive predictive value for the learning results of 8p23.1 duplication syndrome, and the results verified using the model obtained from this learning are shown as test data.

Figure 6 shows the sensitivity and positive predictive value for the learning results of 17q21.31 duplication syndrome, and the results verified using the model obtained from this learning are shown as test data.

**Mode for Carrying out the Invention**

Hereinafter, the present invention will be described in more detail by way of the following examples. However, the following examples are for illustrating the present invention, and the scope of the present invention is not limited to these examples.

**Example 1. Non-invasive detection of fetal copy number variation based on synthetic data production**

**1. Preparation of cell-free DNA and DNA library for DNA sequencing**

Blood from a total of 15,999 pregnant women with fetuses of the negative samples was collected. Approximately 10 mL of peripheral blood was drawn from the subjects and collected in a BCT™ tube (Streck, Omaha, NE, USA). The collected blood samples were centrifuged at 1,200 x g at 4°C for 15 minutes. Blood plasma was collected and centrifuged again at 16,000 x g at 4°C for 10 minutes. Cell-free DNA (cfDNA) was obtained from the centrifuged plasma using the MagListo™ cfDNA extraction kit (Bioneer, Republic of Korea).

The obtained cfDNA fragment was end-repaired using T4 DNA polymerase, Klenow DNA polymerase, and T4 polynucleotide kinase, and the cfDNA fragment was obtained again using the Agencourt AMPure XP (Fisher Scientific).

A DNA library for the ion proton sequencing system was constructed from the prepared cfDNA according to the protocol provided by the manufacturer (ThermoFisher Scientific). Using the 540 chip, an average 0.3x sequencing coverage depth per nucleotide was calculated.

**2. Massive parallel sequencing**

The DNA library prepared as described in 1. was subjected to massive parallel sequencing using the Ion Torrent S5XL™ system (ThermoFisher Scientific).

Different raw reads were obtained using Ion Torrent Suite™ software (ThermoFisher Scientific). The filtered reads were aligned to the human genome reference sequence hg19 by Burrows-Wheeler transform (BWT). Sequence reads that were mapped to only one genomic location in hg19 were aligned as unique reads. Of the total reads, about $3.3 \times 10^6$ were unique reads, and the GC content of the total 15,999 samples ranged from about 39% to 45%.

Among the 15,999 samples described in 1., 530 male fetus samples and 530 female fetus samples were selected, which had the unique read count of 2 million or more, the GC content of 39.5% or more and less than 42%, and the fetal DNA fraction of 4% or more. The fetal DNA fraction was determined using SNP genotyping and imputation method (Korean Patent Registration No. KR 10-2031841) for the selected samples. At this time, the MAF filtering criterion value was set to 7%, and the correlation coefficient with the fetal DNA fraction based on chromosome Y was 98%.

In order to produce synthetic data, male fetuses and female fetuses were not separated from each other, and two samples were randomly selected from male fetuses and female fetuses, combined, and divided in half, but the two samples were selected so that no two samples overlapped. In other words, two samples were randomly selected from 500 male fetuses and 500 female fetuses, combined, and then divided in half again to produce 160,000 new synthetic learning data (80,000 negative, 80,000 positive). In addition, two samples were randomly selected from 30 male fetuses and 30 female fetuses, combined, and then divided in half again to produce 20,000 new synthetic test data (10,000 negative, 10,000 positive). Here, when combining into one and dividing in half again, the read count corresponding to 50% of the total read count (RC) was randomly selected. The fetal DNA fraction (fetal fraction: FF) of the synthetic data (C) was calculated by multiplying the AFF (fetal fraction of A) and the total read counts of A (read counts of A: ARC) from the two selected samples (A, B), to obtain the fetal read count of A (ARC × AFF), and multiplying the BFF (fetal fraction of B) and the total read counts of B (read counts of B: BRC), to obtain the fetal read count of B (BRC × BFF), and then adding them up and dividing them by the sum of the total read counts of A and B. In other words, the FF of the synthetic data (C) may be calculated by the equation of FF = (ARC × AFF + BRC × BFF) / (ARC + BRC).

### 3. Production and detection of synthetic positive data with copy number variation from synthetic normal data

### A. Method of producing synthetic positive data with deletion copy number variation from synthetic normal chromosome data

The method of producing a chromosome with deletion copy number variation from a normal chromosome is to subtract the result of multiplying the read count of the corresponding normal chromosome site by 50% of the fetal DNA fraction (FF) from the read count (RC) of the chromosome site where the corresponding copy number variation exists. In other words, this can be expressed as an equation: DRCPC = RCPC - RCPC × FF/2. In the above equation, DRCPC (read count of a part of chromosome in a deletion) is the read count in the target chromosome site in a copy number variation fetus, RCPC (read count of a part of chromosome) is the read count in the target chromosome site in a normal fetus, and FF is the fetal DNA fraction.

A specific method of producing a deletion copy number variation by targeting a specific site of a normal chromosome is explained using the example of chromosome 1 short arm (p-arm) 36 deletion (1p36 deletion syndrome).

When any one of samples is selected and 1p36 is targeted, the DNA reads of this site are mixed with maternal and fetal reads. At this time, it is important to know the ratio of the DNA reads in a fetus as accurately as possible. Therefore, in the case of a male fetus and a female fetus, the fetal DNA fraction (FF) using the SNP-based imputation method is used.

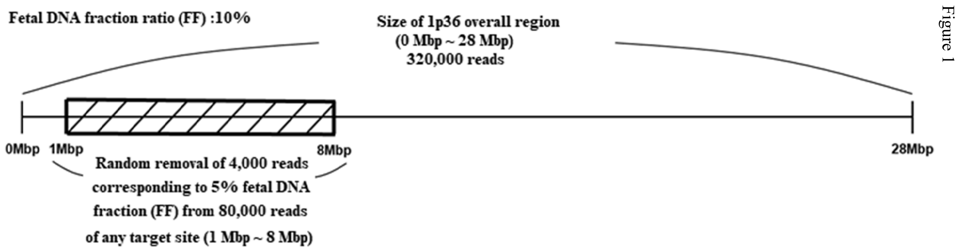

In the case of a normal fetus, the number of DNA reads assigned to 1p36 is determined by deriving from a pair of maternal chromosomes and a pair of fetal chromosomes. Here, as shown in Figure 1, the size of the overall region corresponding to 1p36 is 28 Mbp, and the size of the target site is randomly selected at an any location within 0 Mbp to 28 Mbp. Therefore, the DNA read count of a target site having an any size corresponding to the p36 region of chromosome 1 of a normal fetus is determined by multiplying the total DNA read count assigned to the target site by the fetal DNA fraction (FF). On the other hand, since the copy number variation with 1p36 deletion exists in an any target site within the 1p36 region by a partial deletion of a chromosome of a specific size, 50% must be additionally subtracted from the normal fetal DNA read count of that any target site. As a result, when the DNA read count assigned to an any target site within the p36 region of chromosome 1 in an any normal fetal sample is RCPC, the chromosome deletion site (DRCPC) of the 1p36 deletion sample may be calculated by the equation of DRCPC = RCPC - RCPC × FF/2.

Using the same method, 5p deletion and 9p deletion can also be applied and obtained.

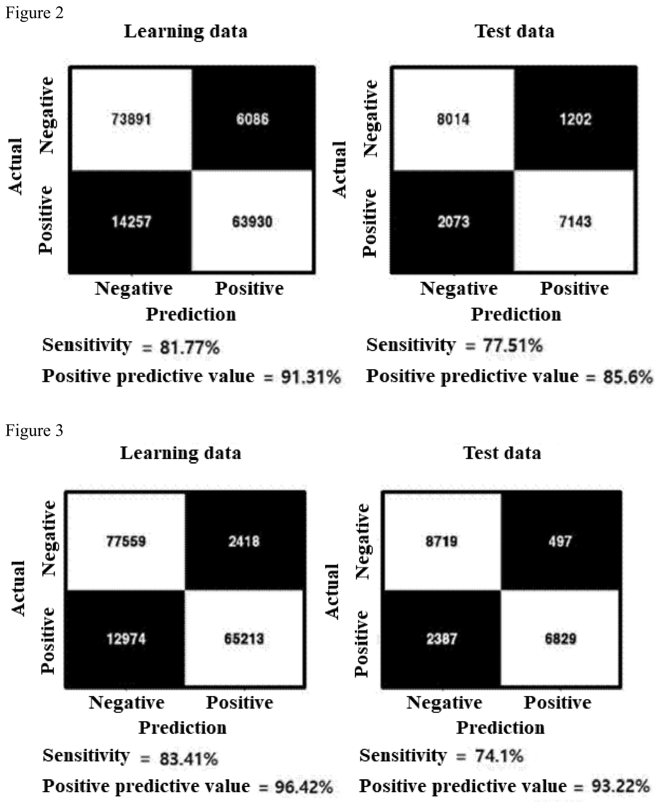

Figures 2 to 4 show the results of learning with about 160,000 data (learning data) (80,000 negative, 80,000 positive) and using about 20,000 verification data (test data) (10,000 negative, 10,000 positive).

As shown in Figure 2, a model was established by learning (learning data) with a logistic regression algorithm using a total of 98 parameters consisting of RCPC, RCPCi (i = 1 to i = 95, section size: 300 Kb, i is the number of sections into which the chromosome site is divided evenly, and the total number of sections k = 95), FF, and GC content, using about 80,000 synthetic normal data and about 80,000 synthetic positive data. Here, RCPC is normal and positive data, and the deletion size of the positive data was 1 Mbp or more, and FF was 4% or more. In addition, the sensitivity and positive predictive value of the test sample (test data) verified using synthetic data (10,000 normal, 10,000 positive) were calculated, and the calculated results are shown in Figure 2. As shown in Figure 2, in the case of 1p36 deletion syndrome, it was confirmed that the sensitivity in the learning data was about 81.8%, the positive predictive value was about 91.3%, and the sensitivity and positive predictive value in the test data were about 77.5% and about 85.6%, respectively.

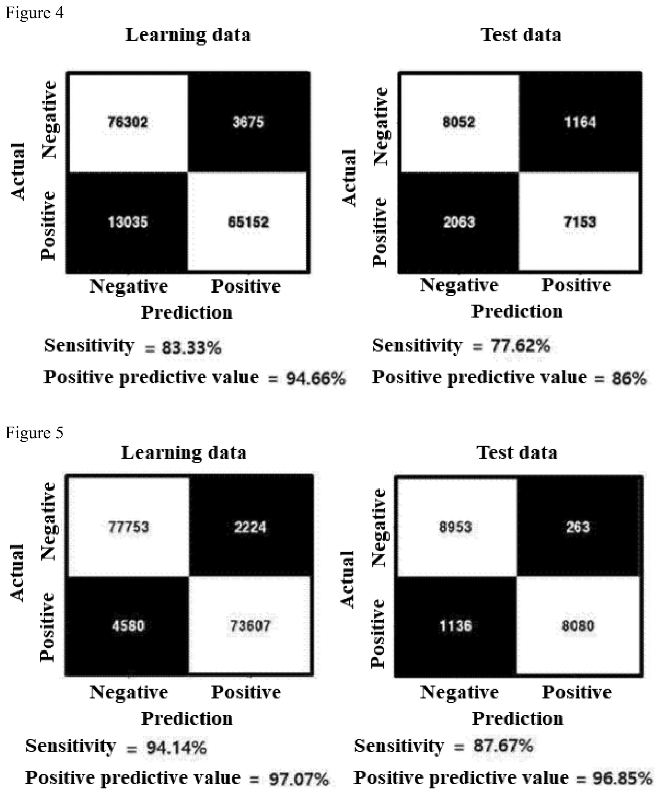

In Figure 3, 5p deletion syndrome was shown, and a model was established by learning (learning data) with a logistic regression algorithm using a total of 164 parameters consisting of RCPC, RCPCi (i = 1 to i = 161, section size: 300 Kb, i; the number of sections into which the chromosome site is divided evenly, and the total number of sections k = 161), FF, and GC content, using 80,000 synthetic normal data and 80,000 synthetic positive data. Here, RCPC is normal and positive data, and the deletion size of the positive data was 1 Mbp or more, and FF was 4% or more. In addition, the sensitivity and positive predictive value of the test sample (test data) verified using synthetic data (10,000 normal, 10,000 positive) were calculated, and the calculated results are shown in Figure 3. As shown in Figure 3, in the case of 5p deletion syndrome, it was confirmed that the sensitivity in the learning data was about 83.4%, the positive predictive value was about 96.4%, and the sensitivity and positive predictive value in the test data were about 74.1% and about 93.22%, respectively.

In Figure 4, 9p deletion syndrome was also shown in the same manner, and since RCPCi (i = 1 to i = 163) was different, a total of 166 parameters were used for learning and testing. Here, RCPC is normal and positive data, and the deletion size of the positive data was 1 Mbp or more, and FF was 4% or more. In addition, the sensitivity and positive predictive value of the test sample (test data) verified using synthetic data (10,000 normal, 10,000 positive) were calculated, and the calculated results are shown in Figure 4. As shown in Figure 4, in the case of 9p deletion syndrome, it was confirmed that the sensitivity in the learning data was about 83.3%, the positive predictive value was about 94.7%, and the sensitivity and positive predictive value in the test data were about 77.6% and about 86%, respectively.

**B. Method of producing synthetic positive data with duplication copy number variation from synthetic normal chromosome data**

The method of producing a chromosome with duplication copy number variation from a normal chromosome is add the read count (RC) of the chromosome site where the corresponding copy number variation exists plus the result of multiplying the read count of the corresponding normal chromosome site by 50% of the fetal DNA fraction (fetal fraction: FF). In other words, this can be expressed as an equation: DRCPC = RCPC + RCPC × FF/2. In the above equation, DRCPC (read count of a part of chromosome in a duplication) is the read count in the target chromosome site in a copy number variation fetus, RCPC (read count of a part of chromosome) is the read count in the target chromosome site in a normal fetus, and FF is the fetal DNA fraction.

A specific method of producing a duplication copy number variation by targeting a specific site of a normal chromosome is explained using the example of chromosome 8 short arm (p-arm) 23.1 duplication (8p23.1 duplication syndrome).

When any one of samples is selected and 8p23.1 is targeted, the DNA reads of this site are mixed with maternal and fetal reads. At this time, it is important to know the ratio of the DNA reads in a fetus as accurately as possible. Therefore, in the case of a male fetus and a female fetus, the fetal DNA fraction (FF) using the SNP-based imputation method is used.

In the case of a normal fetus, the number of DNA reads assigned to 8p23.1 is determined by deriving from a pair of maternal chromosomes and a pair of fetal chromosomes. Here, the size of the overall region corresponding to 8p23.1 is 6.5 Mbp, and the size of the target site is randomly selected at an any location within 0 Mbp to 6.5 Mbp. Therefore, the DNA read count of a target site having an any size corresponding to the p23.1 region of chromosome 8 of a normal fetus is determined by multiplying the total DNA read count assigned to the target site by the fetal DNA fraction (FF). On the other hand, since the copy number variation with duplication exists in an any target site within the 8p23.1 region by a partial duplication of a chromosome of a specific size, 50% must be additionally added to the normal fetal DNA read count of that any target site. As a result, when the DNA read count assigned to an any target site within the p23.1 region of chromosome 8 in an any sample is RCPC, the copy number variation (DRCPC) of the 8p23.1 duplication sample may be calculated by the equation of DRCPC = RCPC + RCPC × FF/2.

Using the same method, 17q21.31 (chromosome 17 long arm (q-arm) 21.31 site) duplication can also be applied and obtained.

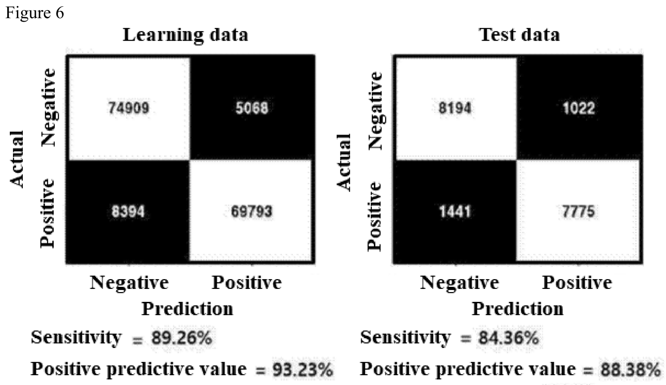

Figures 5 and 6 show the results of learning with about 160,000 data (learning data) (80,000 negative, 80,000 positive) and using about 20,000 verification data (test data) (10,000 negative, 10,000 positive).

As shown in Figure 5, 8p23.1 duplication syndrome was shown. A model was established by learning (learning data) with a logistic regression algorithm using a total of 24 parameters consisting of RCPC, RCPCi (i = 1 to i = 21, section size: 300 Kb, the total number of sections k = 21), FF, and GC content, using 80,000 synthetic normal data and 80,000 synthetic positive data. Here, RCPC is normal and positive data, and the deletion size of the positive data was 1 Mbp or more, and FF was 4% or more. In addition, the sensitivity and positive predictive value of the test sample (test data) verified using synthetic data (10,000 normal, 10,000 positive) were calculated, and the calculated results are shown in Figure 5. As shown in Figure 5, in the case of 8p23.1 duplication syndrome, it was confirmed that the sensitivity in the learning data was about 94.1%, the positive predictive value was about 97.1%, and the sensitivity and positive predictive value in the test data were about 87.7% and about 96.9%, respectively.

In Figure 6, 17q21.31 duplication syndrome was shown. A model was established by learning (learning data) with a logistic regression algorithm using a total of 17 parameters consisting of RCPC, RCPCi (i = 1 to i = 14, section size: 300 Kb, the total number of sections k = 14), FF, and GC content, using 80,000 synthetic normal data and 80,000 synthetic positive data. Here, RCPC is normal and positive data, and the deletion size of the positive data was 1 Mbp or more, and FF was 4% or more. In addition, the sensitivity and positive predictive value of the test sample (test data) verified using synthetic data (10,000 normal, 10,000 positive) were calculated, and the calculated results are shown in Figure 6. As shown in Figure 6, in the case of 17q21.31 duplication syndrome, it was confirmed that the sensitivity in the learning data was about 89.3%, the positive predictive value was about 93.2%, and the sensitivity and positive predictive value in the test data were about 84.4% and about 88.4%, respectively.

**Claims**

1. A method for detecting fetal copy number variations, comprising:

    obtaining sequence information (read) of a plurality of nucleic acid fragments obtained from a biological sample of a pregnant woman with a normal fetus;
    mapping the obtained sequence information to a human reference genome and aligning the sequence information of the nucleic acid fragments to a chromosome;
    calculating the read count (RC), fetal DNA fraction (fetal fraction: FF), and GC content of the sequence information of the nucleic acid fragments in the target chromosome in a normal fetus, based on the sequence information of the nucleic acid fragments aligned to the chromosome;
    producing synthetic normal data from normal data based on the read count and fetal DNA fraction in the target chromosome in a normal fetus, wherein the normal data is the read count in the target chromosome site in a normal fetus;
    producing synthetic positive data from the synthetic normal data, wherein the synthetic positive data is the read count in the target chromosome site in a fetus with copy number variations;
    establishing a copy number variation discrimination model from the produced synthetic normal data and synthetic positive data; and
    detecting copy number variations of a test sample using the copy number variation discrimination model.

2. The method according to claim 1, wherein the target chromosome site is a site of one selected from the group consisting of chromosome 1 to chromosome 22, chromosome X, and chromosome Y.

3. The method according to claim 2, wherein the target chromosome site is a site of one selected from the group consisting of chromosome 1, chromosome 5, chromosome 8, chromosome 9, and chromosome 17.

4. The method according to claim 3, wherein the target chromosome site is selected from the group consisting of chromosome 1 short arm 36 site (1p36), chromosome 5 short arm site (5p), chromosome 8 short arm 23.1 site (8p23.1), chromosome 9 short arm site (9p), and chromosome 17 long arm 21.31 site (17q21.31).

5. The method according to claim 1, wherein the copy number variation is any one variation selected from the group consisting of insertion, deletion, duplication, inversion, and translocation.

6. The method according to claim 5, wherein the copy number variation is selected from the group consisting of 1p36 deletion, 5p deletion, 8p23.1 duplication, 9p deletion, and 17q21.31 duplication.

7. The method according to claim 1, wherein the copy number variation causes one of the disorders selected from 1p36 deletion syndrome, 5p deletion syndrome (Cri-du-chat syndrome), 8p23.1 duplication syndrome, 9p deletion syndrome, 11q deletion syndrome (Jacobsen syndrome), 17q21.31 duplication syndrome, autism spectrum disorder, epilepsy, schizophrenia, TAR (thrombocytopenia-absent radius) syndrome, HNPP syndrome, 3q29 microdeletion syndrome, 8p23.1 deletion syndrome, Sotos syndrome, Langer-Giedion syndrome, WAGR syndrome (Wilms tumour-aniridia syndrome, aniridia-Wilms tumour syndrome), Koolen-de Vries syndrome, Beckwith-Wiedemann syndrome, DiGeorge syndrome, Charcot-Marie-Tooth disease, Miller-Dieker Lissencephaly syndrome, Angelman syndrome, Williams syndrome, Smith-Magenis syndrome, Prader-Willi syndrome, De Grouchy syndrome, Xp11.2 duplication syndrome, and Wolf-Hirschhorn syndrome, Mowat-Wilson syndrome, Mesomelia-Synostoses syndrome, Kleefstra syndrome, Kagami-Ogata syndrome, Alagille syndrome, Okihiro syndrome, Aldred syndrome, Syndactyl-Nystagmus syndrome, Silver-Russell syndrome, and Yuan-Harel-Lupski syndrome.

8. The method according to claim 1, wherein producing synthetic positive data from the synthetic normal data is determined by the following equation when the copy number variation of the target chromosome site is a deletion:

$$DRCPC = RCPC - RCPC \times FF/2,$$

in the above equation,

DRCPC is the read count in the target chromosome site in a copy number variation fetus,
RCPC is the read count in the target chromosome site in a normal fetus, and
FF is the fetal DNA fraction.

9. The method according to claim 1, wherein producing synthetic positive data from the synthetic normal data is determined by the following equation when the copy number variation of the target chromosome site is a duplication:

$$DRCPC = RCPC + RCPC \times FF/2,$$

in the above equation,

DRCPC is the read count in the target chromosome site in a copy number variation fetus,
RCPC is the read count in the target chromosome site in a normal fetus, and
FF is the fetal DNA fraction.

10. The method according to claim 1, wherein establishing a copy number variation discrimination model from the produced synthetic normal data and synthetic positive data is performed based on RCPC (read count of a part of chromosome), RCPCi (i = 1 to i = k, i is the number of sections into which the chromosome site is divided evenly, and k is the total number of sections), FF, and GC content, or a combination thereof, when the copy number variation of the target chromosome site is a deletion or duplication, wherein RCPC is normal and positive data.

11. The method according to claim 1, wherein detecting copy number variations of a test sample using the copy number variation discrimination model is performed based on RCPC (read count of a part of chromosome), RCPCi (chromosome i = 1 to i = k, i is the number of sections into which the chromosome site is divided evenly, and k is the total number of sections), FF, and GC content, or a combination thereof, when the copy number variation of the target chromosome site is a deletion or duplication, wherein RCPC is normal and positive data.

12. The method according to claim 1, wherein the copy number variation discrimination model is trained by applying a machine learning algorithm.

13. The method according to claim 12, wherein the machine learning algorithm is selected from the group consisting of LGBM (Light Gradient Boosting Machine), AdaBoost, Voting Classifier, Random Forest, logistic regression analysis (logistic algorithm), artificial neural network, and QDA (Quadratic Discriminant Analysis).

14. A computer-readable medium recording a program applied to perform the method according to any one of claims 1 to 13.

Figure 1

Fetal DNA fraction ratio (FF) :10%

Size of 1p36 overall region
(0 Mbp ~ 28 Mbp)
320,000 reads

0Mbp 1Mbp 8Mbp 28Mbp

Random removal of 4,000 reads
corresponding to 5% fetal DNA
fraction (FF) from 80,000 reads
of any target site (1 Mbp ~ 8 Mbp)

Figure 2

**Learning data**

Sensitivity = 81.77%

Positive predictive value = 91.31%

**Test data**

Sensitivity = 77.51%

Positive predictive value = 85.6%

Figure 3

**Learning data**

Sensitivity = 83.41%

Positive predictive value = 96.42%

**Test data**

Sensitivity = 74.1%

Positive predictive value = 93.22%

Figure 4

**Learning data**

Sensitivity = 83.33%

Positive predictive value = 94.66%

**Test data**

Sensitivity = 77.62%

Positive predictive value = 86%

Figure 5

**Learning data**

Sensitivity = 94.14%

Positive predictive value = 97.07%

**Test data**

Sensitivity = 87.67%

Positive predictive value = 96.85%

Figure 6

## INTERNATIONAL SEARCH REPORT

| International application No. |
|---|
| **PCT/KR2023/018626** |

### A. CLASSIFICATION OF SUBJECT MATTER

**G16B 20/20**(2019.01)i; **G16B 30/10**(2019.01)i; **G16B 40/20**(2019.01)i; **G16B 20/30**(2019.01)i; **G16B 10/00**(2019.01)i; **G16B 45/00**(2019.01)i; **G06N 3/02**(2006.01)i; **G06N 20/00**(2019.01)i

According to International Patent Classification (IPC) or to both national classification and IPC

### B. FIELDS SEARCHED

Minimum documentation searched (classification system followed by classification symbols)

G16B 20/20(2019.01); C12Q 1/68(2006.01); G06F 19/18(2011.01); G06F 19/22(2011.01); G06N 3/08(2006.01); G16B 20/10(2019.01); G16B 25/10(2019.01); G16B 30/00(2019.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 복제수 변이(copy number variation), 삽입(insertion), 결실(deletion), 중복(duplication), 역위(inversion), 전좌(translocation), 맵핑(mapping), 리드수(read count), 태아 DNA 분획(fetal DNA fraction), GC 함량(GC content), 가상 양성 데이터(false-positive data), 가상 정상 데이터(false-normal data), 모델(model)

### C. DOCUMENTS CONSIDERED TO BE RELEVANT

| Category* | Citation of document, with indication, where appropriate, of the relevant passages | Relevant to claim No. |
|---|---|---|
| Y | KR 10-2017-0088780 A (GENOMECARE CO., LTD.) 02 August 2017 (2017-08-02)<br>See claims 1 and 4; and paragraphs [0038], [0041]-[0049] and [0086]-[0095]. | 1-14 |
| Y | KR 10-2021-0058888 A (THE JACKSON LABORATORY) 24 May 2021 (2021-05-24)<br>See claims 1, 7, 10 and 13-14; paragraphs [0036] and [0049]; and table 1. | 1-14 |
| A | KR 10-2021-0121941 A (SEOUL NATIONAL UNIVERSITY HOSPITAL et al.) 08 October 2021 (2021-10-08)<br>See entire document. | 1-14 |
| A | KR 10-1686146 B1 (GREEN CROSS GENOME CORPORATION) 13 December 2016 (2016-12-13)<br>See entire document. | 1-14 |

☑ Further documents are listed in the continuation of Box C.     ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **04 March 2024** | **04 March 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

| International application No. |
|---|
| **PCT/KR2023/018626** |

| C. | DOCUMENTS CONSIDERED TO BE RELEVANT | |
|---|---|---|
| Category* | Citation of document, with indication, where appropriate, of the relevant passages | Relevant to claim No. |
| A | US 2021-0327535 A1 (THE REGENTS OF THE UNIVERSITY OF CALIFORNIA et al.) 21 October 2021 (2021-10-21)<br>See entire document. | 1-14 |
| A | KR 10-2021-0067931 A (GREEN CROSS GENOME CORPORATION) 08 June 2021 (2021-06-08)<br>See entire document. | 1-14 |

Form PCT/ISA/210 (second sheet) (July 2022)

## INTERNATIONAL SEARCH REPORT
### Information on patent family members

International application No.

**PCT/KR2023/018626**

| Patent document cited in search report | | | Publication date (day/month/year) | Patent family member(s) | | | Publication date (day/month/year) |
|---|---|---|---|---|---|---|---|
| KR | 10-2017-0088780 | A | 02 August 2017 | KR | 10-1881098 | B1 | 24 July 2018 |
| KR | 10-2021-0058888 | A | 24 May 2021 | CN | 112955959 | A | 11 June 2021 |
| | | | | EP | 3850631 | A1 | 21 July 2021 |
| | | | | US | 2022-0059185 | A1 | 24 February 2022 |
| | | | | WO | 2020-056302 | A1 | 19 March 2020 |
| KR | 10-2021-0121941 | A | 08 October 2021 | KR | 10-2385528 | B1 | 13 April 2022 |
| KR | 10-1686146 | B1 | 13 December 2016 | | None | | |
| US | 2021-0327535 | A1 | 21 October 2021 | CN | 113574602 | A | 29 October 2021 |
| | | | | EP | 3841583 | A1 | 30 June 2021 |
| | | | | EP | 3841583 | A4 | 18 May 2022 |
| | | | | WO | 2020-041611 | A1 | 27 February 2020 |
| | | | | WO | 2020-041611 | A8 | 11 March 2021 |
| KR | 10-2021-0067931 | A | 08 June 2021 | AU | 2020-391556 | A1 | 23 June 2022 |
| | | | | AU | 2020-391556 | B2 | 04 January 2024 |
| | | | | CA | 3163405 | A1 | 03 June 2021 |
| | | | | EP | 4068291 | A1 | 05 October 2022 |
| | | | | JP | 2023-504139 | A | 01 February 2023 |
| | | | | KR | 10-2586651 | B1 | 11 October 2023 |
| | | | | US | 2023-0028790 | A1 | 26 January 2023 |
| | | | | WO | 2021-107676 | A1 | 03 June 2021 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 2031841 B

- KR 102031841

**Non-patent literature cited in the description**

- **GUOLIN KE et al.** LightGBM: A Highly Efficient Gradient Boosting Decision Tree. *Proceedings of the 31st International Conference on Neural Information Processing Systems (NIPS 2017)*, 3149-3157